# EUROPÄISCHE PATENTSCHRIFT

(11) **EP 0 343 430 B1**
(45) Veröffentlichungstag und Bekanntmachung des Hinweises auf die Patenterteilung: **22.09.1993**
(21) Anmeldenummer: 89108404.8
(22) Anmeldetag: 10.05.1989
(51) Int. Cl.: G03B 42/02, H05G 1/64, H05G 1/02, A61B 6/02

(54) **Lichtverteiler für eine Röntgendiagnostikeinrichtung**
Light distributor for an X-ray diagnostic installation
Distributeur de lumière pour installation de diagnostic à rayons X

(30) Priorität: 25.05.1988 DE 8806828 U
(43) Veröffentlichungstag der Anmeldung: 29.11.1989
(73) Patentinhaber: SIEMENS AKTIENGESELLSCHAFT, 80333 München (DE)
(72) Erfinder: Gall, Arthur, Dipl.-Ing., D-8521 Langensendelbach (DE); Kütterer, Gerhard,Dipl.-Ing. (FH), D-8520 Erlangen (DE); Richter, Helmut, D-8523 Baiersdorf (DE)

(56) Entgegenhaltungen:
- EP-A- 0 051 430
- EP-A- 0 052 995
- EP-A- 0 175 663
- DE-U- 8 707 204
- DE-U- 8 806 828
- US-A- 3 622 786
- US-A- 3 684 354

## Beschreibung

Die Erfindung betrifft einen Lichtverteiler für eine Röntgendiagnostikeinrichtung gemäß dem Oberbegriff des Patentanspruches 1. Derartige Lichtverteiler ermöglichen den seriellen oder parallelen Betrieb von verschiedenen Aufnahmegeräten, wie beispielsweise eine Fernsehkamera, eine Kino- und eine Blattfilm- oder Kleinbildkamera, zur Aufnahme von Ausgangsbildern eines Röntgenbildverstärkers.

In der US-PS 4 383 328 ist ein derartiger Lichtverteiler beschrieben, der das Ausgangsbild des Röntgenbildverstärkers auf zwei Kinokameras und eine Fernsehkamera verteilt. Ein klappbar angebrachter, halbdurchlässiger Spiegel bewirkt die Aufteilung auf die zwei Kinokameras. Dieser Klappspiegel bleibt aber in seiner Parkstellung innerhalb des Strahlenganges einer Kinokamera. Vor dem klappbaren Spiegel in seiner Ruhestellung läßt sich ein weiterer Spiegel mit einer Reflexion von 100 % einschieben, der senkrecht zum Strahlengang des Röntgenbildverstärkers angeordnet ist. Eine Spiegelanordnung mit einem um 45° gekippten, drehbaren Spiegel mit einer Reflexion von 90 % läßt sich im Strahlengang vor dem einschiebbaren Spiegel einfahren, der eine wahlweise Ablenkung des Strahlenganges auf eine der Kinokameras oder eine Fernsehkamera ermöglicht. Es wurde zwar bei diesem Lichtverteiler Wert darauf gelegt, einen möglichst kompakten Aufbau zu erreichen, damit die Scheitelabstände der Linsen der Kinokameras zu der dem Röntgenbildverstärker zugeordneten Basisoptik zur Erzielung einer guten Bildqualität klein gehalten werden können. Dies ist deshalb wichtig, da die Lichtverluste mit dem Abstand der Scheitel der Linsen wachsen. Da aber die Spiegel hintereinander in verschiedenen Ebenen in den Strahlengang eingefahren werden können, erhöht sich der Abstand in unerwünschter Weise. Weiterhin ist es schwierig, klappbare Spiegel ausreichend stabil gegen Erschütterungen während des Betriebes zu halten. Auch muß ein derartiger Lichtverteiler mehrere Motoren zur Betätigung der Spiegel aufweisen, so daß er einen komplizierten Aufbau und ein relativ hohes Gewicht aufweist. Ferner erhöht sich die Baulänge der Röntgenbildverstärker-Aufnahmeeinheit in unerwünschter Weise, da eine der Aufnahmekomponenten, beispielsweise die erste Kinokamera, sich in dem direkten Strahlengang des Röntgenbildverstärkers befindet. Dadurch reicht in einigen Fällen die Raumhöhe des Untersuchungsraumes bei der Verwendung von Untertischgeräten und einem zusätzlichen Deckenstativ nicht aus.

In der DE-U-87 07 204 ist ein Lichtverteiler beschrieben, bei dem ein oder mehrere Schlitten vorgesehen sind, die in Führungsstangen verfahrbar sind, die senkrecht auf dem Strahlengang der Basisoptik stehen. Auf diesen Schlitten sind Spiegel angebracht, deren Flächen gegeneinander sowie zu den Schlitten unterschiedliche Winkel aufweisen. Dadurch ist es zwar möglich, den Strahlengang auf mehrere Komponenten umzulenken. Durch den winkligen Aufbau jedoch ergeben sich auch hier große Scheitelabstände der Linsen, so daß unerwünschte Lichtverluste entstehen.

Die Erfindung geht von der Aufgabe aus, einen Lichtverteiler gemäß dem Oberbegriff des Patentanspruches 1 zu schaffen, der einen stabilen und kompakten Aufbau, so daß die Scheitelabstände der Linsen klein gehalten werden können, und ein geringes Gewicht aufweist.

Die Aufgabe wird erfindungsgemäß durch die kennzeichnenden Merkmale des Patentanspruches 1 gelöst. Durch diese schräge Anordnung in einer Ebene tragen die Spiegelflächen sowie ihre Befestigungen wenig zur Länge des Scheitelabstandes bei, so daß ein sehr kompakter und kleiner Lichtverteiler erhalten wird.

Einen besonders kompakten Aufbau erhält man, wenn die Führungen parallel zu einer zweiten Ebene liegen, die durch den auftreffenden und den durch die Spiegelflächen abgelenkten Strahlengang gebildet wird. Dadurch trägt die relativ lange Anordnung der beiden neben- bzw. hintereinanderliegenden Spiegel wenig zur Vergrößerung der Bauform bei, da die Spiegelflächen im wesentlichen zwischen den an den Lichtverteiler angekoppelten Komponenten geführt wird.

Einen Mehrkanal-Lichtverteiler erhält man, wenn im Strahlengang eine weitere bewegliche Spiegelfläche angeordnet ist, die dreh- oder schwenkbar mit dem Gehäuse verbunden ist. Als vorteilhaft hat es sich dabei erwiesen, wenn die weitere bewegliche Spiegelfläche im Strahlengang hinter den beiden in einer Ebene verschiebbaren Spiegelflächen angeordnet ist. Eine stabile und leicht zu justierende Anordnung der beweglichen Spiegelflächen erhält man, wenn für die Führungen zwei Führungsstangen vorgesehen sind, und wenn der Schlitten auf der einen Seite Kugelbüchsen aufweist, die eine erste der beiden Führungsstangen umschließen, und wenn er über Rollager mit einer zweiten der beiden Führungsstangen verbunden ist. Der Schlitten für die beweglichen Spiegelflächen läßt sich vorteilhaft verschieben, wenn der Schlitten mit einem einseitig am Gehäuse befestigten Kurbeltrieb verbunden ist, der mit einem Drehantrieb versehen ist. Eine optimalere, platzsparendere Anbringung der Aufnahmekomponenten erhält man, wenn wenigstens eine der Optiken eine Winkel-Optik ist, die eine seitliche Ablenkung des Strahlenganges um einen Winkel bewirkt. Eine kostengünstigere Variante ergibt sich, wenn die Basis-Optik eine Geradeaus-Optik ist und wenn ein fester Spiegel im Gehäuse des Lichtverteilers befestigt ist, der eine seitliche Ablenkung des Strahlenganges um einen Winkel bewirkt. Zusätzlich läßt sich Bauhöhe einsparen, wenn der Winkel ca. 80° beträgt. Die Fernsehkamera läßt sich vorteilhaft durch die ersten Spiegel ankoppeln, wenn der teildurchlässige Spiegel eine Reflexion von ca. 20 % aufweist.

Einen Vierkanal-Lichtverteiler mit einem kurzen Scheitel-Abstand für eine angekoppelte Fernsehkamera und mit drei Kamerakanälen mit jeweiliger Fernseh-Mitbeobachtung erhält man, wenn in dem Strahlengang der Basis-Optik die zwei auf dem Schlitten verschiebbar befestigte Spiegelflächen angeordnet sind, die wenigstens einen Teil des von der Basis-Optik fallenden Lichtes auf eine an dem Lichtverteiler angekoppelten Fernsehkamera ablenken, und wenn den Spiegelflächen eine weitere drehbare Spiegelfläche nachgeordnet ist, die in drei Stellungen gehalten werden kann, so daß der parallele Strahlengang des Ausgangsbildes des Röntgenbildverstärkers alternativ auf drei an dem Gehäuse des Lichtverteilers befestigte Aufnahmekomponente gelenkt werden kann. Einen optimalen Fernsehbetrieb bei wahlweiser Mitbeobachtung erhält man, wenn eine der Speigelflächen eine Reflexion von 100 % und die andere, teildurchlässige, eine Reflexion von 20 % aufweist.

Die Erfindung ist nachfolgend anhand von in der Zeichnung dargestellten Ausführungsbeispielen näher erläutert. Es zeigen:
- Fig. 1: eine Röntgendiagnostikeinrichtung mit einem Lichtverteiler nach dem Stand der Technik,
- Fig. 2: eine Darstellung eines erfindungsgemäßen Zweikanal-Lichtverteilers aus der Sicht des Röntgenbildverstärkers,
- Fig. 3: eine Draufsicht auf den erfindungsgemäßen Zweikanal-Lichtverteiler gemäß Fig. 2,
- Fig. 4: eine schematische Darstellung einer weiteren Ausführungsform eines erfindungsgemäßen Zweikanal-Lichtverteilers, und
- Fig. 5: eine schematische Darstellung eines erfindungsgemäßen Mehrkanal-Lichtverteilers.

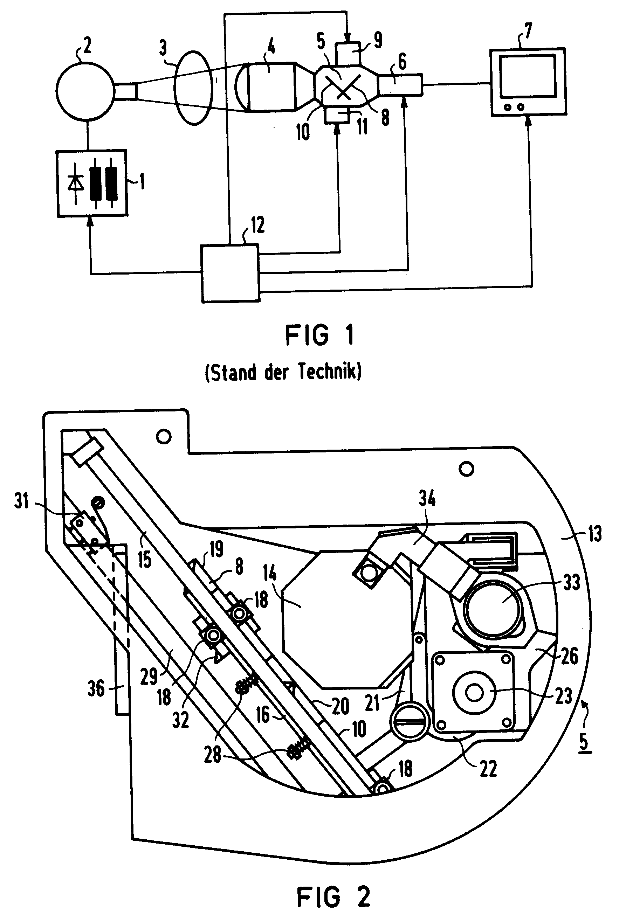

In der Fig. 1 ist eine Röntgendiagnostikeinrichtung nach dem Stand der Technik mit einem Hochspannungsgenerator 1 dargestellt, der eine Röntgenröhre 2 betreibt. Die Röntgenröhre 2 sendet ein Strahlenbündel aus, das einen Patienten 3 durchdringt und auf den Eingangsleuchtschirm eines Röntgenbildverstärkers 4 fällt. An dem Ausgang des Röntgenbildverstärkers 4 ist ein Lichtverteiler 5 angekoppelt, an dessen einem Ausgang eine Fernsehkamera 6 als eine erste Aufnahmekomponente befestigt ist, die das Ausgangsbild des Röntgenbildverstärkers 4 auf einem Monitor 7 darstellt. Durch einen ersten beweglichen, in den Strahlengang des Röntgenbildverstärkers 4 einbringbaren Spiegel 8 wird das Ausgangsbild des Röntgenbildverstärkers 4 auf beispielsweise eine Kinokamera 9 als eine zweite Aufnahmekomponente abgelenkt. Ein zweiter beweglicher, ebenfalls in den Strahlengang einbringbarer Spiegel 10 lenkt den Strahlengang in eine andere Richtung ab, so daß das Ausgangsbild des Röntgenbildverstärkers 4 beispielsweise von einer Fotokamera 11 als eine dritte Aufnahmekomponente aufgenommen werden kann. Eine Zentraleinheit 12 bewirkt dabei die Steuerung und Synchronisation des Hochspannungsgenerators 1, der Fernsehkette 6 und 7, der Spiegel 8 und 10 sowie der Kameras 9 und 11.

Wird eine derartige Röntgendiagnostikeinrichtung als Untertischgerät eingesetzt, bei dem die Röntgenröhre 2 unterhalb des Patientenlagerungstisches und die Bildverstärker-Aufnahmeeinheit 4 bis 11 oberhalb des Patientenlagerungstisches angeordnet ist, ergibt sich die maximal benötigte Raumhöhe aus der Höhe der vollständig hochgefahrenen Röntgenbildverstärker-Aufnahmeeinheit 4 bis 11. Die vom Röntgenbildverstärker 4 wegweisende Fernsehkamera 6 erhöht dabei den maximalen Raumbedarf in unerwünschter Weise.

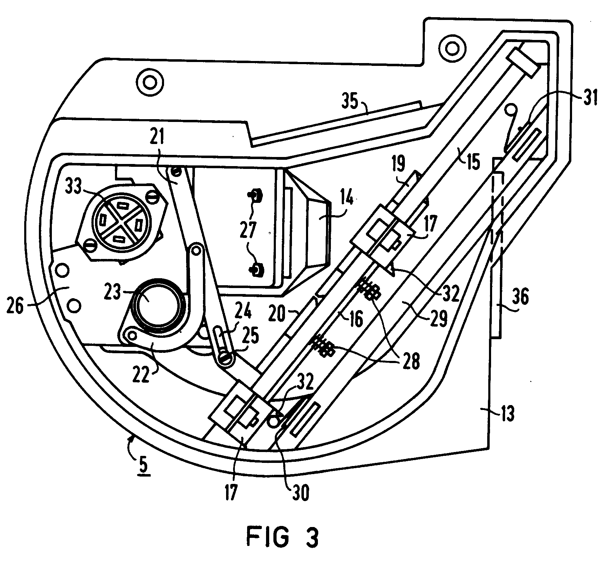

In der Fig. 2 ist ein Gehäuse 13 eines erfindungsgemäßen Lichtverteilers 5 von unten aus der Sicht des Röntgenbildverstärkers 4 dargestellt. In dem Strahlengang des Röntgenbildverstärkers hinter einer nicht dargestellten Basis-Optik, die in bekannter Weise einen parallelen Strahlengang erzeugt, befindet sich innerhalb des Gehäuses 13 ein erster Spiegel 14, der den Strahlengang nahezu rechtwinklig auf eine an dem Gehäuse 13 durch eine Halterung 36 ankuppelbare fotografische Kamera 9 bzw. 11 als zweite Aufnahmekomponente lenkt. Dieser erste Spiegel 14 kann fest mit dem Gehäuse 13 verbunden sein. Den Strahlengang zwischen dem ersten Spiegel 14 und der ankuppelbaren Aufnahmekomponente umgeben zwei Führungsstangen 15, auf denen ein Schlitten 16 beweglich gehalten ist. Der Schlitten 16 weist, wie aus Fig. 3 ersichtlich, die eine Draufsicht des in Fig. 2 dargestellten Lichtverteilers 5 wiedergibt, Kugelbüchsen 17 auf, die eine der Führungsstangen 15 umgreifen, während die andere Seite mit Rollen 18 versehen ist, die die zweite Führungsstange 15 als Rollager beidseitig umfassen, so daß der Schlitten 16 entlang der Führungsstangen 15 verfahrbar ist. Auf dem Schlitten 16 sind zwei Spiegel 8 und 10 als bewegliche Spiegelflächen 19 und 20 befestigt. Diese zwei beweglichen Spiegelflächen 19 und 20 lassen sich durch einen Kurbeltrieb in den Strahlengang zwischen erstem Spiegel 14 und fotografischer Kamera 9 bzw. 11 verfahren, so daß die Spiegelflächen 19 und 20 den Strahlengang auf eine an dem Gehäuse 13 an einer Halterung 35 ankuppelbare Fernsehkamera 6 als erste Aufnahmekomponente lenkt.

Die erste Spiegelfläche 19 kann einen Reflexionsgrad von 100 % aufweisen, so daß in der dargestellten Stellung die erste Spiegelfläche 19 den gesamten Lichtanteil des parallelen Strahlenganges in die Optik der angekoppelten Fernsehkamera 6 leitet, so daß bis auf geringe Verluste das vom Ausgangsleuchtschirm des Röntgenbildverstärkers 4 ausgehende Licht auf das Target der Fernsehkamera 6 geleitet wird.

Die zweite bewegliche Spiegelfläche 20 kann einen Reflexionsgrad von ca. 20 % aufweisen, so daß ein großer Teil des auf sie auftreffenden Lichtes durchgelassen wird, während nur ein kleiner Teil reflektiert wird. Wird nun, wie noch später beschrieben wird, der Schlitten 16 entlang der Führungstangen 15 derart verfahren, daß sich der zweite bewegliche Spiegel 10 mit seiner Spiegelfläche 20 im vom Spiegel 14 herkommenden parallelen Strahlengang befindet, so wird durch diese zweite Spiegelfläche 20 80% des vom Ausgangsleuchtschirm des Röntgenbildverstärker ausgehenden Lichtes auf die angekoppelte photographische Kamera 9 bzw. 11 durchgelassen. 20% des Ausgangslichtes des Röntgenbildverstärkers 4 dagegen wird auf die Fernsehkamera 6 reflektiert, so daß bei gleichzeitiger Beobachtung des Ausgangsbildes des Röntgenbildverstärkers 4 durch die Fernsehkamera 6 eine Aufnahme durch die photographische Kamera 9 bzw. 11 erfolgen kann.

Der Kurbeltrieb umfaßt eine Kurbelschwinge 21, einen bogenförmig gekrümmten Arm 22 und einen Drehantrieb 23, beispielsweise einen Motor. Die Kurbelschwinge 21 ist einseitig an dem Gehäuse 13 befestigt. Auf der anderen Seite weist die Kurbelschwinge 21 ein Langloch 24 auf, in das ein an dem Schlitten 16 befestigter Bolzen 25 eingreift. Der Drehantrieb 23 ist über einen ersten Träger 26 mit dem Gehäuse 13 verbunden. Die Spiegel 8, 10 und 14 lassen sich durch Justierschrauben 27 und 28 einstellen.

Unter dem Schlitten 16 ist ein zweiter Träger 29 an dem Gehäuse 13 befestigt, der zwei Mikroschalter 30 und 31 trägt, die als Endschalter für die beweglichen Spiegel 8 und 10 dienen. Hierzu sind die Kugelbüchsen 17 des Schlittens 16 mit Nasen 32 versehen, die die Mikroschalter 30 und 31 in den Endstellungen des Schlittens 16 betätigen.

An dem ersten Träger 26 ist weiterhin ein Fotomultiplier 33 befestigt, dem über einen Lichtleiter 34 ein kleiner Teil des Lichtes des Strahlenganges des Röntgenbildverstärkers 4 vor dem ersten Spiegel 14 seitlich ausgekoppelt wird, so daß in bekannter Weise die Helligkeit des Ausgangsbildes des Röntgenbildverstärkers 4 und damit die Röntgenröhrenstrahlung geregelt werden können.

Durch diese erfindungsgemäße Anordnung erhält man einen äußerst kompakten Lichtverteiler 5, der den Strahlengang des Röntgenbildverstärkers 4 jeweils auf die seitlich angebrachten Aufnahmekomponenten 6, 9 umlenkt, so daß sich nur eine geringe Höhe des Lichtverteilers 5 mit angekoppelten Aufnahmekomponenten 6, 9 ergibt. Gleichzeitig weist er eine stabile Spiegelanordnung auf. Ebenfalls enthält er nur wenige einfache Steuermittel, so daß der Lichtverteiler 5 nur ein geringes Gewicht aufweist und wenig störanfällig ist.

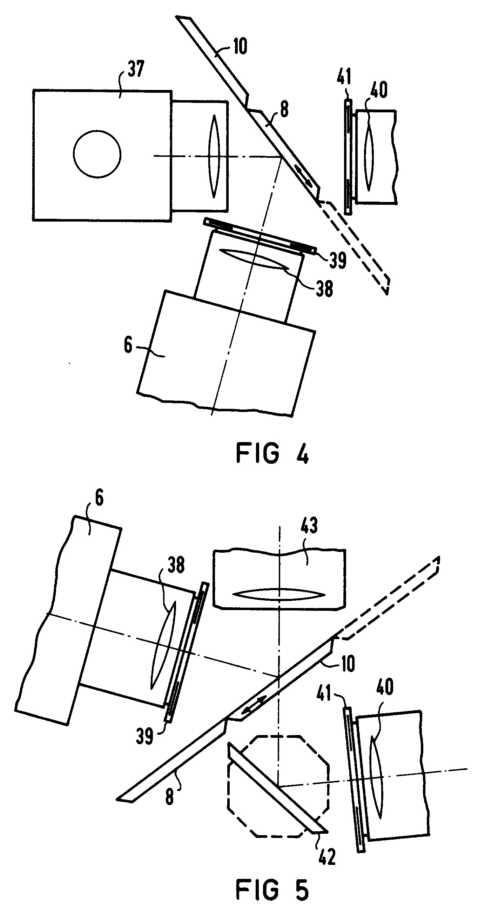

In FIG 4 ist eine weitere Ausführungsform des erfindungsgemäßen Lichtverteilers 5 schematisch dargestellt. Anstelle des ersten Spiegels 14 und der Geradeaus-Optik als Basis-Optik wird nun eine Winkel-Optik 37 verwendet, so daß sich die Abmessung in Richtung der optischen Achse des Röntgenbildverstärkers 4 weiter reduziert, da die Längsabmessungen der Winkel-Optik 37 geringer sind als die der Basis-Optik zusammen mit dem Spiegel 14. Der erste Spiegel 8 leitet den Strahlengang der Winkel-Optik 37 in die Optik 38 der Fernsehkamera 6, der eine Irisblende 39 vorgeschaltet ist. Werden nun die Spiegel 8 und 10 in die gestrichelt dargestellte Stellung verfahren, so daß der zweite Spiegel 10 sich im Strahlgang befindet, so wird ein großer Teil des Lichtes des Strahlenganges in die Optik 40 der photographischen Kamera 9 bzw. 11 geleitet. Die Lichtmenge auch dieser Optik 40 läßt sich durch eine Irisblende 41 einstellen. Der kleinere Teil des Lichtes des parallelen Strahlenganges wird, wie bereits beschrieben, zur Kontrolle auf die Fernsehkamera 6 umgelenkt.

In FIG. 5 ist schematisch der Aufbau eines erfindungsgemäßes 3- bzw. 4-Kanal-Lichtverteiler dargestellt. Einer dem Röntgenbildverstärker 4 zugeordneten Geradeaus-Optik 43 sind die beiden beweglichen Spiegel 8 und 10 in bekannter Weise nachgeordnet. Das durch den teildurchlässigen zweiten beweglichen Spiegel 10 hindurchgehende Licht fällt auf einen dritten beweglichen Spiegel 42, der das Licht seitlich in die Optik 40 beispielsweise einer Kinokamera 9 leitet. Durch Drehung des dritten beweglichen Spiegel 42 um eine Achse, die in Richtung des auf ihn auftreffenden parallelen Strahlenganges verläuft, in die gestrichelt dargestellte Stellung, läßt sich das durch den zweiten beweglichen Spiegel 10 hindurch getretene Licht beispielsweise senkrecht in die Zeichenebene oder senkrecht aus der Zeichenebene ablenken, so daß das Licht wahlweise auf drei verschiedene photographische Kameras geleitet werden kann. Durch die Verwendung der Geradeaus-Optik 43 und den drei beweglichen Spiegeln 8, 10 und 42 sind die Anlagekomponenten 6, 9 und 11 alle in einer Ebene angeordnet, die senkrecht zur in Längsrichtung verlaufenden Mittelachse des Röntgenbildverstärkers ausgerichtet ist, so daß auch in diesem Fall die Abmessung in Längsrichtung gering gehalten werden können.

## Patentansprüche

1. Lichtverteiler für eine Röntgendiagnostikeinrichtung, die einen Röntgenbildverstärker (4) und an dem Lichtverteiler (5) anschließbare Aufnahmekomponenten (6, 9, 11) mit je einer vorgeschalteten Optik (38, 40) aufweist, mit einem Gehäuse (13), mit einer eingangsseitigen, dem Röntgenbildverstärker (4) zugewandten Basis-Optik (37, 43) zur Erzeugung eines parallelen Strahlenganges des Ausgangsbildes des Röntgenbildverstärkers (4), mit Spiegelflächen (19, 20), die auf einem entlang den parallelen Strahlengang umgebenden Führungen (15) verschiebbar angebrachten Schlitten (16) zur wahlweisen Ablenkung des parallelen Strahlenganges auf die an dem Gehäuse (13) anschließbaren Aufnahmekomponenten (6, 9, 11) befestigt sind, und mit Antriebsmitteln (21 bis 25) zur Verschiebung jeweils einer der Spiegelflächen (19, 20) seitlich in den Strahlengang, **dadurch gekennzeichnet,** daß die Führungen (15) in einer Ebene verlaufen, die schräg zum parallelen Strahlengang der Basis-Optik (37, 43) liegt, daß wenigstens zwei der Spiegelflächen (19, 20) nebeneinander flach so auf dem Schlitten (16) befestigt sind, daß sie parallel zur Ebene der Führungen (15) angeordnet sind, und daß wenigstens eine der wenigstens zwei Spiegelflächen (19, 20) teildurchlässig ist.

2. Lichtverteiler nach Anspruch 1, **dadurch gekennzeichnet,** daß die Führungen (15) parallel zu einer zweiten Ebene liegen, die durch den auftreffenden und den durch die Spiegelflächen abgelenkten Strahlengang gebildet wird.

3. Lichtverteiler nach Anspruch 1 oder 2, **dadurch gekennzeichnet,** daß im Strahlengang eine weitere Spiegelfläche (42) angeordnet ist, die dreh- oder schwenkbar mit dem Gehäuse verbunden ist.

4. Lichtverteiler nach Anspruch 3, **dadurch gekennzeichnet,** daß die weitere bewegliche Spiegelfläche (42) im Strahlengang hinter den beiden in einer Ebene verschiebbaren Spiegelflächen (19, 20) angeordnet ist.

5. Lichtverteiler nach einem der Ansprüche 1 bis 4, **dadurch gekennzeichnet,** daß für die Führungen (15) zwei Führungsstangen (15) vorgesehen sind, daß der Schlitten (16) auf der einen Seite Kugelbüchsen (17) aufweist, die eine erste der beiden Führungsstangen (15) umschließen, und daß er über Rollager (18) mit einer zweiten der beiden Führungsstangen (15) verbunden ist.

6. Lichtverteiler nach den Ansprüchen 1 bis 5, **dadurch gekennzeichnet,** daß der Schlitten (16) mit einem einseitig am Gehäuse (13) befestigten Kurbeltrieb (21 bis 25) verbunden ist, der mit einem Drehantrieb (23) versehen ist.

7. Lichtverteiler nach einem der Ansprüche 1 bis 6, **dadurch gekennzeichnet,** daß wenigstens eine der Optiken (37, 38, 40) eine Winkel-Optik ist, die eine seitliche Ablenkung des Strahlenganges um einen Winkel bewirkt.

8. Lichtverteiler nach einem der Ansprüche 1 bis 6, **dadurch gekennzeichnet,** daß die Basis-Optik eine Geradeaus-Optik (43) ist und daß ein fester Spiegel (14) im Gehäuse (13) des Lichtverteilers (5) befestigt ist, der eine seitliche Ablenkung des Strahlenganges um einen Winkel bewirkt.

9. Lichtverteiler nach Anspruch 7 oder 8, **dadurch gekennzeichnet,** daß der Winkel 80° beträgt.

10. Lichtverteiler nach einem der Ansprüche 1 bis 9, **dadurch gekennzeichnet,** daß die teildurchlässige Spiegelfläche (19, 20) eine Reflexion von 20% aufweist.

11. Lichtverteiler nach einem der Ansprüche 1 bis 10, **dadurch gekennzeichnet,** daß in dem Strahlengang der Basis-Optik (37, 43) die zwei auf dem Schlitten (16) verschiebbar befestigten Spiegelflächen (19, 20) angeordnet sind, die wenigstens einen Teil des von der Basis-Optik (37, 43) fallenden Lichtes auf eine an dem Lichtverteiler (5) angekoppelte Fernsehkamera (6) ablenken, und daß den Spiegelflächen (19, 20) eine weitere drehbare Spiegelfläche (42) nachgeordnet ist, die in drei Stellungen gehalten werden kann, so daß der parallele Strahlengang des Ausgangsbildes des Röntgenbildverstärkers (4) alternativ auf drei an dem Gehäuse (13) des Lichtverteilers (5) befestigte Aufnahmekomponenten (9, 11) gelenkt werden kann.

## Claims

1. Light distributor for an X-ray diagnostic installation which has an X-ray image intensifier (4) and pick-up components (6, 9, 11), connectable to the light distributor (5), with a respective optical system (38, 40) previously connected thereto, having a housing (13), having a base optical system (37, 43), which is on the input side and which faces the X-ray image intensifier (4), for generating a parallel beam path of the output image of the X-ray image intensifier (4), having reflector surfaces (19, 20), which are secured to a carriage (16), mounted such that it can be displaced along guides (15) which surround the parallel beam path, for selective deflection of the parallel beam path onto the pick-up components (6, 9, 11) which can be connected to the housing (13), and having drive means (21 to 25) for the displacement of, in each case, one of the reflector surfaces (19, 20) laterally into the beam path, characterised in that the guides (15) extend in a plane which lies obliquely in relation to the parallel beam path of the base optical system (37, 43), in that at least two of the reflector surfaces (19, 20) are secured side by side in a planar manner on the carriage (16) in such a way that they are arranged parallel to the plane of the guides (15) and in that at least one of the at least two reflector surfaces (19, 20) is semi-transparent.

2. Light distributor according to claim 1, characterised in that the guides (15) lie so as to be parallel to a second plane which is formed by the impinging beam path and the beam path which is deflected by the reflector surfaces.

3. Light distributor according to claim 1 or 2, characterised in that a further reflector surface (42) is arranged in the beam path, which reflector surface is connected with the housing in a rotatable or pivoted manner.

4. Light distributor according to claim 3, characterised in that the further movable reflector surface (42) is arranged in the beam path behind the two reflector surfaces (19, 20) which can be displaced in one plane.

5. Light distributor according to one of the claims 1 to 4, characterised in that two guide rods (15) are provided for the guides (15), in that the carriage (16) has on the one side ball boxes (17) surrounding a first guide rod of the two guide rods (15) and in that it is connected by way of roller bearings (18) with a second guide rod of the two guide rods (15).

6. Light distributor according to claims 1 to 5, characterised in that the carriage (16) is connected with a crank mechanism (21 to 25) which is secured on the one side to the housing (13) and which is provided with a rotary drive (23).

7. Light distributor according to one of the claims 1 to 6, characterised in that at least one of the optical systems (37, 38, 40) is an angular optical system which effects a lateral deflection of the beam path about an angle.

8. Light distributor according to one of the claims 1 to 6, characterised in that the base optical system is a straight-line optical system (43) and in that a fixed reflector (14) is secured in the housing (13) of the light distributor (5) which effects a lateral deflection of the beam path about an angle.

9. Light distributor according to claim 7 or 8, characterised in that the angle amounts to 80°.

10. Light distributor according to one of the claims 1 to 9, characterised in that the semi-transparent reflector surface (19, 20) has a reflection of 20%.

11. Light distributor according to one of the claims 1 to 10, characterised in that the two reflector surfaces (19, 20), which are secured to the carriage (16) in a displaceable manner and which deflect at least a portion of the light which falls from the base optical system (37, 43) onto a television camera (6) coupled to the light distributor (5), are arranged in the beam path of the base optical system (37, 43) and in that arranged after the reflector surfaces (19, 20) there is a further rotatable reflector surface (42) which can be held in three positions so that the parallel beam path of the output image of the X-ray image intensifier (4) can be alternatively directed onto three pick-up components (9, 11) which are secured to the housing (13) of the light distributor (5).

## Revendications

1. Répartiteur de lumière pour une installation de radiodiagnostic, qui comporte un amplificateur de brillance radiologique (4) et des composants de réception (6,9,11) qui peuvent être raccordés au répartiteur de lumière (5) et en amont de chacun est branché un système optique (38,40), et comportant un boîtier (12), un système optique de base (37,43) situé côté entrée, associé à l'amplificateur de brillance radiologique (4) et servant à produire un trajet de rayonnement parallèle de l'image issue de l'amplificateur de brillance radiologique (4), les surfaces réfléchissantes (19,20), qui sont fixées sur un chariot (16) qui est monté de manière à être déplaçable le long de guides (15) entourant le trajet de rayonnement parallèle, pour dévier au choix le trajet de rayonnement en parallèle en direction des composants de réception (6,9,11) pouvant être raccordés au boîtier (13), et comportant des moyens d'entraînement (21 à 25) servant à déplacer respectivement latéralement l'une des surfaces réfléchissantes (19,20) pour l'amener dans le trajet du rayonnement, caractérisé par le fait que les guides (15) s'étendent dans un plan qui est disposé obliquement par rapport au trajet de rayonnement parallèle du système optique de base (37,40), qu'au moins deux des surfaces réfléchissantes (19,20) sont fixées à plat l'une à côté de l'autre sur le chariot (16) de telle sorte qu'elles sont parallèles au plan des guides (15) et qu'au moins l'une des au moins deux surfaces réfléchissantes (19,20) est partiellement transparente.

2. Répartiteur de lumière suivant la revendication 1, caractérisé par le fait que les guides (15) sont parallèles à un second plan qui est formé par le trajet du rayonnement incident et le trajet du rayonnement dévié par les surfaces réfléchissantes.

3. Répartiteur de lumière suivant la revendication 1 ou 2, caractérisé par le fait que dans le trajet du rayonnement est disposée une autre surface réfléchissante (42), qui est raccordée au boîtier de manière à pouvoir tourner ou basculer.

4. Répartiteur de lumière suivant la revendication 3, caractérisé par le fait que l'autre surface réfléchissante mobile (42) est disposée dans le trajet du rayonnement en arrière des deux surfaces réfléchissantes (19,20) déplaçables dans un plan.

5. Répartiteur de lumière suivant l'une des revendications 1 à 4, caractérisé par le fait que pour les guides (15) il est prévu deux barres de guidage (15), que le chariot (16) possède, d'un côté, des douilles à billes (17), qui enserrent une première des deux barres de guidage (15), et que le chariot est relié par l'intermédiaire de roulements à rouleaux (18) à une seconde des deux barres de guidage (4).

6. Répartiteur de lumière suivant les revendications 1 à 5, caractérisé par le fait que le chariot (16) est raccordé à un dispositif d'entraînement à manivelle (21 à 25), qui est fixé unilatéralement au boîtier (13) et comporte un dispositif d'entraînement en rotation (23).

7. Répartiteur de lumière suivant l'une des revendications 1 à 6, caractérisé par le fait qu'au moins l'un des systèmes optiques (37,38,40) est un système optique angulaire qui réalise une déviation latérale du trajet du faisceau sur un certain angle.

8. Répartiteur de lumière suivant l'une des revendications 1 à 6, caractérisé par le fait que le système de base est un système optique rectiligne (43) et qu'un miroir fixe (14) est fixé dans le boîtier (13) du répartiteur de lumière (5), ce miroir réalisant une déviation latérale du faisceau de rayonnement sur un certain angle.

9. Répartiteur de lumière suivant la revendication 7 ou 8, caractérisé par le fait que l'angle est égal à 80°.

10. Répartiteur de lumière suivant l'une des revendications 1 à 9, caractérisé par le fait que la surface partiellement transparente (19,20) du miroir produit une réflexion de 20 %.

11. Répartiteur de lumière suivant l'une des revendications 1 à 10, caractérisé par le fait que dans le trajet du rayonnement du système optique de base (37,42) sont disposées les deux surfaces réfléchissantes (19,20), qui sont fixées de manière à être déplaçables sur le chariot (16) et qui dévient au moins une partie de la lumière provenant du système optique de base (37,43), en direction d'une caméra de télévision (6) couplée au répartiteur de lumière (5), et qu'en aval des surfaces réfléchissantes (19,20) est disposée une autre surface réfléchissante rotative (42), qui peut être maintenue dans trois positions, de sorte que le faisceau de rayonnement parallèle de l'image de sortie de l'amplificateur de brillance radiologique (4) peut être dévié alternativement vers trois composants de réception (9,11) fixés au boîtier (13) du répartiteur de lumière (5).
